# EUROPEAN PATENT SPECIFICATION

(11) **EP 3 931 147 B1**
(45) Date of publication and mention of the grant of the patent: **07.06.2023**
(21) Application number: 20707427.9
(22) Date of filing: 27.02.2020
(51) Int. Cl.: C01B 3/38, C01B 3/48

(54) **CHEMICAL PLANT WITH A REFORMING SECTION AND A PROCESS FOR PRODUCING A CHEMICAL PRODUCT**
CHEMISCHE ANLAGE MIT EINEM REFORMIERUNGSABSCHNITT UND VERFAHREN ZUR HERSTELLUNG EINES CHEMISCHEN PRODUKTS
INSTALLATION CHIMIQUE DOTÉE D'UNE SECTION DE REFORMAGE ET PROCÉDÉ DE PRODUCTION D'UN PRODUIT CHIMIQUE

(30) Priority: 28.02.2019 DK PA201900258; 15.07.2019 DK PA201900873
(43) Date of publication of application: 05.01.2022
(73) Proprietor: Topsoe A/S, 2800 Kgs. Lyngby (DK)
(72) Inventor: MORTENSEN, Peter Mølgaard, 4000 Roskilde (DK); AASBERG-PETERSEN, Kim, 3450 Allerød (DK)
(74) Representative: Topsoe A/S
(86) International application number: PCT/EP2020/055173
(87) International publication number: WO 2020/174056

(56) References cited:
- WO-A1-2013/013895
- WO-A1-2014/180888
- GB-A- 2 448 089
- US-A- 3 450 507
- US-A1- 2015 175 416
- US-A1- 2016 115 017
- US-B1- 6 390 030

## Description

### FIELD OF THE INVENTION

Embodiments of the invention generally relate to a chemical plant including a reforming section for reforming a feed gas comprising hydrocarbons to a synthesis gas and to a process for reforming a feed gas comprising hydrocarbons to a synthesis gas.

### BACKGROUND

Processes based on Autothermal Reforming (ATR) is a route to production of synthesis gas. The main elements of an ATR reactor are a burner, a combustion chamber, and a catalyst bed contained within a refractory lined pressure shell. In an ATR reactor, partial combustion of the hydrocarbon feed by sub-stoichiometric amounts of oxygen is followed by steam reforming of the partially combusted hydrocarbon feed stream in a fixed bed of steam reforming catalyst. Steam reforming also takes place to some extent in the combustion chamber due to the high temperature. The steam reforming reaction is accompanied by the water gas shift reaction. Typically, the gas is at or close to equilibrium at the outlet of the reactor with respect to steam reforming and water gas shift reactions. The temperature of the exit gas is typically in the range between 850° and 1100°C. More details of ATR and a full description can be found in the art such as "Studies in Surface Science and Catalysis, Vol. 152," Synthesis gas production for FT synthesis"; Chapter 4, p.258-352, 2004".

US 3 450 507 A discloses a reformer comprising a plurality of primary reformer tubes externally heated by any suitable means, such as combustion of a fluid hydrocarbon or using electric resistor heating elements.

It is also an object of the invention to provide a system for the production of a synthesis gas where the cost for the equipment materials (CAPEX) is reduced compared to known systems for production of similar amounts of synthesis gas using ATRs. Furthermore, it is an object of the invention to provide a process for synthesis gas production using an ATR with a reduced CO₂ footprint compared to known processes using ATRs, for similar amounts of synthesis gas produced.

It is an object of the invention to provide a process and system for production of a synthesis gas by use of an ATR wherein the size and duty of the fired heater upstream the ATR is reduced, or ideally removed, compared to known processes and systems using ATRs.

It is a further object of the invention to provide a process and system for production of a synthesis gas by use of an ATR wherein the system can be started up quickly.

### SUMMARY OF THE INVENTION

An aspect of the invention relates to chemical plant comprising:
- a reforming section arranged to receive a feed gas comprising hydrocarbons and provide an output synthesis gas, wherein said reforming section comprises:
   - an electrically heated reforming reactor housing a first catalyst, said electrically heated reforming reactor being arranged for receiving said feed gas and generating a first synthesis gas,
   - an autothermal reforming reactor downstream said electrically heated reforming reactor, said autothermal reforming reactor housing a second catalyst, said autothermal reforming reactor being arranged for receiving said first synthesis gas and outputting a second synthesis gas, wherein said reforming section is arranged to output said output synthesis gas comprising said second synthesis gas as from claim 1.

When the reforming section of the chemical plant comprises an electrically heated reforming reactor upstream an autothermal reforming reactor, quick and efficient heating of the feed gas takes place in the electrically heated reforming reactor together with steam reforming. The first synthesis gas exiting the electrically heated reforming reactor is heated sufficiently for autothermal reforming to take place in the autothermal reforming reactor. Typically, the temperature of the first synthesis gas exiting the electrically heated reforming reactor has a temperature of at least about 350-400°C, such as above 400°C, preferably above 450°C, more preferably above 500°C, and more preferably above even 600°C. Thereby, it is typically not necessary to heat the first synthesis gas typically between the electrically heated reforming reactor and the autothermal reforming reactor.

A chemical plant with an autothermal reforming reactor typically includes a fired heater unit arranged to preheat the feed gas prior to entering the autothermal reforming reactor. When the chemical plant comprises an electrical reforming reactor upstream the autothermal reforming reactor, the fired heater unit may be reduced considerably in size or it may even be omitted.

When the reforming section comprises an electrically heated reforming reactor upstream an autothermal reforming reactor, the chemical energy of the first synthesis gas inlet into the autothermal reforming reactor may be increased considerably in comparison with a feed gas that has been heated in a fired heater or in another type of heater, such as an electrical heater, and subsequently preformed in an adiabatic prereformer followed by final heating before entering the autothermal reforming reactor. Due to the increased chemical energy, the required amount of oxygen inlet into the autothermal reforming reactor is potentially reduced. Here, the term "chemical energy of a gas" is meant to denote the enthalpy or internal energy of the gas, so that a gas has a higher chemical energy if it has higher enthalpy and/or a higher internal energy state.

It should be noted that part of the feed gas may bypass the electrically heated reforming reactor so that it is sent directly to the autothermal reforming reactor or is mixed with the first synthesis gas upstream the autothermal reforming reactor.

Moreover, the term "steam reforming" or "steam methane reforming reaction" is meant to denote a reforming reaction according to one or more of the following reactions:

CH₄ + H₂O ↔ CO + 3H₂ (i)

CH₄ + 2H₂O ↔ CO₂ + 4H₂ (ii)

CH₄ + CO₂ ↔ 2CO + 2H₂ (iii)

Reactions (i) and (ii) are steam methane reforming reactions, whilst reaction (iii) is the dry methane reforming reaction.

For higher hydrocarbons, viz. CₙHₘ, where n≥2, m ≥ 4, equation (i) is generalized as:

CₙHₘ + n H₂O ↔ nCO + (n + m/2)H₂ (iv)

where n≥2, m ≥ 4.

Typically, steam reforming is accompanied by the water gas shift reaction (v):

CO + H₂O ↔ CO₂ + H₂ (v)

The terms "steam methane reforming" and "steam methane reforming reaction" are meant to cover the reactions (i) and (ii), the terms "steam reforming" and "electrically heated reforming" are meant to cover the reactions (i), (ii) and (iv), whilst the term "methanation" covers the reverse reaction of reaction (i). In most cases, all of these reactions (i)-(v) are at, or close to, equilibrium at the outlet from the reforming reactor. The term "prereforming" is often used to cover the catalytic conversion of higher hydrocarbons according to reaction (iv). Prereforming is typically accompanied by steam reforming of methane and/or methanation (depending upon the gas composition and operating conditions) and the water gas shift reaction. Prereforming is often carried out in adiabatic reactors but may also take place in heated reactors.

Moreover, the terms "autothermal reforming" and "autothermal reforming reactions" also covers combustion and partial combustion of the hydrocarbon feedstock according to reaction (vi) and (vii):

CH₄ + 1/2O₂ ↔ CO + 2H₂ (vi)

CH₄ + 2O₂ ↔ CO₂ + 2H₂O (vii)

in addition to the steam methane reforming reactions.

The term "synthesis gas" is meant to denote a gas comprising hydrogen, carbon monoxide and also carbon dioxide and small amounts of other gasses, such as argon, nitrogen, methane, etc.

The term "prereforming" is often used to cover the catalytic conversion of higher hydrocarbons, viz. CₙHₘ, where n≥2, m ≥ 4, according to the reaction:

CₙHₘ + n H₂O ↔ nCO + (n + m/2)H₂

Prereforming is typically accompanied by steam reforming of methane and/or methanation (depending upon the gas composition and operating conditions) and the water gas shift reaction. Prereforming is often carried out in adiabatic reactors but may also take place in heated reactors. Prereforming typically takes place in a temperature range of ca. 350-550°C to convert higher hydrocarbons as an initial step in the process. This removes the risk of carbon formation from higher hydrocarbons on catalyst and equipment in the subsequent process steps. Optionally, carbon dioxide or other components may also be mixed with the gas leaving the prereforming step to form the feed gas.

A reforming section with an electrically heated reforming reactor upstream an autothermal reforming reactor is advantageous compared a reforming section with a fired steam methane reforming reactor upstream an autothermal reforming reactor, at least due to the following reasons:
- The overall fuel consumption to the reforming section is reduced since no hydrocarbon gas is combusted within a fired steam methane reformer in order to provide heat to the electrically heated reforming reactor;
- a lower accumulated generation of carbon dioxide compared to the combination of a fired steam methane reformer and an autothermal reforming reactor, in particular when the electrical power for the electrically heated reforming reactor is from renewable sources,
- The overall emission of carbon dioxide and other emissions detrimental to the climate or the local environment, such as NOₓ or SOₓ, are reduced considerably by minimizing the amount of hydrocarbons used for providing heat for the reforming reactions;
- The reactor footprint is reduced strongly due to the compactness of the electrically heated reformer.

Moreover, a reforming section with an electrically heated reforming reactor upstream an autothermal reforming reactor is advantageous compared a reforming section with a fired heater upstream an autothermal reforming reactor, at least due to the following reasons:
- The chemical plant may be reduced in size since the fired heater upstream the autothermal reforming reactor may be reduced considerably in sized, or even done without; and
- The H₂/CO ratio of the output synthesis gas from the reforming section can be adjusted by controlling the temperature of the first synthesis gas exiting the electrically heated reforming reactor, and thereby indirectly controlling the oxygen consumption.
- The overall emission of carbon dioxide and other emissions detrimental to the climate or the local environment, such as NOₓ or SOₓ, are reduced considerably by minimizing the amount of hydrocarbons used for providing heat for the fired heater.
- The overall oxygen consumption will be reduced because the chemical energy of the first synthesis gas is higher than the chemical energy of a gas exiting a fired heater at comparable temperatures as a result of the chemical reaction, which have taken place in the electrically heated reforming reactor.

Moreover, the plant of the invention has provided a possibility of carrying out the start-up and increase of load of the autothermal reforming reactor quicker as compared to a system with a fired heater unit and an autothermal reforming reactor. The combination of an electrically heated reforming reactor and an autothermal reforming reactor has benefits compared to for example a combination of an electrically heated reforming reactor and a fired steam methane reforming reactor or compared to a combination of an electrical heater followed by a fired steam methane reforming reactor. This is due to the possibility of operating at a lower steam-to-carbon ratio in the plant of the present invention as explained in the following.

The typical outlet temperature from an ATR is in the range 850-1100°C. According to the present invention, the second synthesis gas leaving the ATR has a temperature between 850-1100°C, such as between 950-1075°C. It is known in the art that it is possible to operate an ATR at a steam-to-carbon (S/C) ratio less than 2.0, such as less than 1.5, even lower than 1.2, lower than 1.0, lower than 0.8 or even lower than 0.65. A typical fired steam methane reformer operates at an outlet temperature of 800-950°C. At such a temperature level, the minimum possible S/C-ratio in a fired steam methane reformer will be dictated by the risk of carbon formation on the catalyst in the tubes of the fired steam methane reformer. For these reasons, fired steam methane reformers are not operated at S/C-ratios lower than ca. 1.3-1.5.

The present invention is based on the recognition that the optimum temperature of a gas exiting a prereformer to an ATR is 500-700°C, e.g. 650°C. The invention is further based on the recognition that it is possible to operate an electrically heated reforming reactor at any temperature in a range of about 250-1100°C or even in a broader range and hence at the said optimum temperature, and that therefore it will be highly advantageous to use an electrically heated reforming reactor as a prereformer in combination with an ATR.

The required S/C-ratio to avoid carbon formation on a steam reforming catalyst increases with increasing temperature. As an electrically heated prereformer may be operated at a significantly lower temperature than a fired prereformer, the combination of an electrically heated reforming reactor followed by an autothermal reforming reactor can operate at a significantly lower S/C-ratio than a) a combination of an electrically heated reforming reactor (or an electrical heater) followed by a fired steam methane reformer as well as b) a combination of a fired reforming reactor followed by an autothermal reforming reactor. This means lower steam flows through the process plant and associated reduced costs.

Furthermore, it is possible with the plant of the invention to produce synthesis gas with a reduced H₂/CO-ratio as a result of the reduced steam supply to the process This is desirable for various applications for example if the synthesis gas is used for production of synthetic fuels via the Fischer-Tropsch synthesis.

It should be noted that the terms "steam methane reforming" and "steam reforming" are used as synonyms herein. Moreover, the term "feed gas comprising hydrocarbons" is meant to denote a feed gas comprising a hydrocarbon gas with one or more hydrocarbons and possibly other constituents. The feed gas comprising hydrocarbons fed to the reforming sections is a feed gas comprising hydrocarbons and possibly other constituents, such as carbon monoxide, carbon dioxide, and possibly also some nitrogen and argon, mixed with steam and possibly hydrogen. Examples of "a hydrocarbon gas" may be natural gas, town gas, or a mixture of methane and higher hydrocarbons, as well as a prereformed gas.

Typically, the feed gas comprising hydrocarbons let into the reforming section has a predetermined ratio of hydrocarbon gas, steam, and hydrogen. It should be noted, that a feed gas comprising hydrocarbons to which steam, hydrogen and/or other constituents are added is still denoted a feed gas, even though its composition has changed.

The autothermal reforming reactor is arranged to receive the first synthesis gas and to provide the second synthesis gas, viz. an auto-reformed synthesis gas. In addition to the first synthesis gas input into the autothermal reforming reactor, a stream of oxidant gas is inlet. The stream of oxidant gas comprises oxygen and may be e.g. air or oxygen, enriched air, or a mixture of more than 90% oxygen with the balance being e.g nitrogen, steam, CO₂, and/or argon.

The electrically heated reforming reactor comprises:
- a pressure shell housing an electrical heating unit arranged to heat the first catalyst, where the first catalyst is operable to catalyzing steam reforming of the feed gas, wherein the pressure shell has a design pressure of between 5 and 90 bar,
- a heat insulation layer adjacent to at least part of the inside of the pressure shell, and
- at least two conductors electrically connected to the electrical heating unit and to an electrical power supply placed outside the pressure shell,
wherein the electrical power supply is dimensioned to heat at least part of the first catalyst to a temperature of at least 450°C by passing an electrical current through the electrical heating unit.

In a particular embodiment, the pressure shell has a design pressure of between 5 and 45 bar, preferably between 30 and 45 bar.

A feature of the electrically heated reforming reactor is that the energy is supplied inside the electrically heated reforming reactor, instead of being supplied from an external heat source via heat conduction, convection and radiation, e.g. through catalyst tubes or heater coils. In an electrically heated reforming reactor with an electrical heating unit connected to an electrical power supply via conductors, the heat for the steam reforming reaction is provided by resistance heating. The hottest part of the electrically heated reforming reactor will be within the pressure shell of the electrically heated reforming reactor. Preferably, the electrical power supply and the electrical heating unit within the pressure shell are dimensioned so that at least part of the electrical heating unit reaches a temperature of 450°C-850°C, such as 500°C -750°C, and preferably 550°C -650°C.

The chemical plant of the invention may advantageously comprise one or more compressors and/or pumps upstream the reforming section. The compressors/pumps are arranged to compress the feed to a pressure of between 5 and 90 bar. The constituents of the feed, viz. water/steam, hydrogen and hydrocarbon feed gasses, may be compressed individually and fed individually into the reforming section or to the reforming reactors thereof.

One or more additional feed streams may also be added to the reforming section either upstream the electrically heated reformer, to a location downstream the electrically heated steam reformer and thereby being mixed with the first synthesis gas, or directly to the autothermal reforming reactor. An example is steam added directly to the autothermal reforming reactor or a tail gas from a Fischer-Tropsch unit which for example can be added between the electrically heated reforming reactor and the autothermal reforming reactor or directly to the autothermal reforming reactor. In a Gas-to-Liquids (GTL) plant for production of for example synthetic diesel by Fischer-Tropsch synthesis using natural gas as feedstock, a tail gas is typically recycled from the Fischer-Tropsch synthesis section to the synthesis gas preparation unit. This is done in order to adjust the product composition of the synthesis gas to a desired Hz/CO-ratio of about 2.

The first catalyst may be a bed of catalyst particles, e.g. pellets, typically in the form of catalytically active material supported on a high area support with electrically conductive structures embedded in the bed of catalyst particles. Alternative, the first catalyst may be catalytically active material supported on a macroscopic structure, such as a monolith or a metallic tube.

When the electrically heated reforming reactor comprises a heat insulation layer adjacent to at least part of the inside of the pressure shell, appropriate heat and electrical insulation between the electrical heating unit and the pressure shell is obtained. Typically, the heat insulation layer will be present at the majority of the interior surface of the pressure shell to provide thermal insulation between the pressure shell and the electrical heating unit/first catalyst; however, passages in the heat insulation layers are needed in order to provide for connection of conductors between the electrical heating unit and the electrical power supply and to provide for inlets/outlets for gasses into/out of the electrically heated reforming reactor.

The presence of heat insulating layer between the pressure shell and the electrical heating unit assists in avoiding excessive heating of the pressure shell and assists in reducing thermal losses to the surroundings of the electrically heated reforming reactor. The temperatures of the electrical heating unit may reach up to about 850°C, at least at some parts thereof, but by using the heat insulation layer between the electrical heating unit and the pressure shell, the temperature of the pressure shell can be kept at significantly lower temperatures of e.g. 500°C or even 200°C. This is advantageous since typical construction steel materials are unsuitable or highly expensive for pressure bearing applications at high temperatures, such as 850°C. Moreover, a heat insulating layer between the pressure shell and the electrical heating unit assists in control of the electrical current within the electrically heated reforming reactor, since heat insulation layer is also electrically insulating. The heat insulation layer could be one or more layers of solid material, such as ceramics, inert material, refractory material or a gas barrier or a combination thereof. Thus, it is also conceivable that a purge gas or a confined gas constitutes or forms part of the heat insulation layer.

As the hottest part of the electrically heated reforming reactor during operation is the electrical heating unit, and since a heat insulation layer thermally insulates the pressure shell from the electrically heated reforming reactor, the temperature of the pressure shell can be kept significantly lower than the maximum process temperature. Here, the term "process temperature" is meant to denote the temperature of the gas. This allows for having a relative low design temperature of the pressure shell of e.g. 700°C or 500°C or preferably 300°C or 200°C of the pressure shell whilst having maximum process temperatures of between 750° and 900°, such as 800°, 850°C or 900°C. Another advantage is that the lower design temperature means that in some cases the thickness of the pressure shell can be decreased, thereby saving costs.

It should be noted that the term "heat insulating material" is meant to denote materials having a thermal conductivity of about 10 W·m⁻¹·K⁻¹ or below. Examples of heat insulating materials are ceramics, refractory material, alumina-based materials, zirconiabased materials and similar.

The electrical heating unit comprises a macroscopic structure of electrically conductive material, where the macroscopic structure supports a ceramic coating and the ceramic coating supports the catalytically active material. Such an electrical heating unit is also referred to herein as a "structured catalyst". The catalytic conversion in the form of methane steam reforming and gas heating take place simultaneously.

Thus, during operating of the chemical plant, an electrical current is passed through the macroscopic structure and thereby heats the macroscopic structure and the catalytically active material supported thereon. The close proximity between the catalytically active material and the macroscopic structure enables efficient heating of the catalytically active material by solid material heat conduction from the resistance heated macroscopic structure. The amount and composition of the catalytically active material can be tailored to the steam reforming reaction at the given operating conditions. The surface area of the macroscopic structure, the fraction of the macroscopic structure coated with a ceramic coating, the type and structure of the ceramic coating, and the amount and composition of the catalytically active material may be tailored to the steam reforming reaction at the given operating conditions.

In an embodiment, the structured catalyst has at least one electrically insulating part arranged to increase the current path between the conductors to a length larger than the largest dimension of the structured catalyst. The provision of a current path between the conductors larger than the largest dimension of the structured catalyst may be by provision of electrically insulating part(s) positioned between the conductors and preventing the current running through some part of the structured catalyst. Such electrically insulating parts are arranged to increase the current path and thus increase the resistance through the structured catalyst. Hereby, the current path through the structured catalyst can be e.g. more than 50%, 100%, 200%, 1000%, or even 10000% longer than the largest dimension of the structured catalyst.

The term "electrically conductive" is meant to denote materials with an electrical resistivity in the range from: 10⁻⁴ to 10⁻⁸ Ω·m at 20°C. Thus, materials that are electrically conductive are e.g. metals like copper, silver, aluminum, chromium, iron, nickel, or alloys of metals. Moreover, the term "electrically insulating" is meant to denote materials with an electrical resistivity above 10 Ω·m at 20°C, e.g. in the range from 10⁹ to 10²⁵ Ω·m at 20°C.

As used herein, the term "electrical heating unit comprises a macroscopic catalyst" is not meant to be limited to an electrically heated reforming reactor with a single macroscopic structure. Instead, the term is meant to cover both a macroscopic structure with ceramic coating and catalytically active material supported thereon as well as an array of such macroscopic structures with ceramic coating and catalytically material supported thereon.

The term "macroscopic structure supporting a ceramic coating" is meant to denote that the macroscopic structure is coated by the ceramic coating at, at least, a part of the surface of the macroscopic structure. Thus, the term does not imply that all the surface of the macroscopic structure is coated by the ceramic coating; in particular, at least the parts of the macroscopic structure which are electrically connected to the conductors and thus to the electrical power supply do not have a coating thereon. The coating is a ceramic material with pores in the structure which allows for supporting the catalytically active material of the first catalyst on and inside the coating and has the same function as a catalytic support. Advantageously, the catalytically active material of the first catalyst comprises catalytically active particles having a size in the range from about 5 nm to about 250 nm.

As used herein, the term "macroscopic structure" is meant to denote a structure which is large enough to be visible with the naked eye, without magnifying devices. The dimensions of the macroscopic structure are typically in the range of centimeters or even meters. Dimensions of the macroscopic structure are advantageously made to correspond at least partly to the inner dimensions of the pressure shell, saving room for the heat insulation layer and conductors.

A ceramic coating, with or without catalytically active material, may be added directly to a metal surface by wash coating. The wash coating of a metal surface is a well-known process; a description is given in e.g. Cybulski, A., and Moulijn, J. A., Structured catalysts and reactors, Marcel Dekker, Inc, New York, 1998, Chapter 3, and references herein. The ceramic coating may be added to the surface of the macroscopic structure and subsequently the catalytically active material may be added; alternatively, the ceramic coat comprising the catalytically active material is added to the macroscopic structure.

Preferably, the macroscopic structure has been manufactured by extrusion of a mixture of powdered metallic particles and a binder to an extruded structure and subsequent sintering of the extruded structure, thereby providing a material with a high geometric surface area per volume. A ceramic coating, which may contain the catalytically material, is provided onto the macroscopic structure before a second sintering in an oxidizing atmosphere, in order to form chemical bonds between the ceramic coating and the macroscopic structure. Alternatively, the catalytically active material may be impregnated onto the ceramic coating after the second sintering. When chemical bonds are formed between the ceramic coating and the macroscopic structure, an especially high heat conductivity between the electrically heated macroscopic structure and the catalytically active material supported by the ceramic coating is possible. Due to close proximity between the heat source, viz. the macroscopic structure, and the catalytically active material, the heat transfer is effective, so that the catalytically active material can be very efficiently heated. A compact steam reforming reactor in terms of gas processing per steam reforming reactor volume is thus possible, and therefore the electrically heated reforming reactor housing the macroscopic structure may be compact. The electrically heated reforming reactor of the invention does not need a furnace and this reduces the size of the electrically heated reforming reactor considerably.

According to an embodiment of the system, the macroscopic structure comprises an extruded and sintered monolith or a number of extruded and sintered monoliths electrically connected to each other. According to an embodiment of the system, the macroscopic structure has electrically isolating parts arranged to increase the current path between the conductors to a length larger than the largest dimension of the macroscopic structure.

Preferably, the macroscopic structure comprises Fe, Ni, Cu, Co, Cr, Al, Si or an alloy thereof. Such an alloy may comprise further elements, such as Mn, Y, Zr, C, Co, Mo or combinations thereof. Preferably, the catalytically active material of the first catalyst is particles having a size from 5 nm to 250 nm. The catalytically active material of the first catalyst may e.g. comprise nickel, ruthenium, rhodium, iridium, platinum, cobalt, or a combination thereof. Thus, one possible catalytically active material of the first catalyst is a combination of nickel and rhodium and another combination of nickel and iridium. The ceramic coating may for example be an oxide comprising Al, Zr, Mg, Ce and/or Ca. Exemplary coatings are calcium aluminate or a magnesium aluminum spinel. Such a ceramic coating may comprise further elements, such as La, Y, Ti, K or combinations thereof. Preferably, the conductors are made of different materials than the macroscopic structure. The conductors may for example be of iron, nickel, aluminum, copper, silver, or an alloy thereof. The ceramic coating is an electrically insulating material and will typically have a thickness in the range of around 100 µm, say 10-500 µm. In addition, a fifth catalyst may be placed within the pressure shell and in channels within the macroscopic structure, around the macroscopic structure or upstream and/or downstream the macroscopic structure to support the catalytic function of the macroscopic structure.

In an embodiment, the chemical plant further comprises a prereformer upstream the electrically heated reforming reactor. In the prereformer, the hydrocarbon gas will, together with steam, and potentially also hydrogen and/or other components such as carbon dioxide, undergo prereforming in a temperature range of ca. 350-550°C to convert higher hydrocarbons as an initial step in the process, normally taking place downstream a desulfurization step. This removes the risk of carbon formation from higher hydrocarbons on catalyst in the subsequent process steps. Optionally, carbon dioxide or other components may also be mixed with the gas leaving the prereforming step to form the feed gas.

In an embodiment, the reforming section furthermore comprises a gas heated steam methane reforming reactor in parallel to the electrically heated reforming reactor and the autothermal reforming reactor, wherein the gas heated steam methane reforming reactor comprises a third catalyst and being operable to receive a second feed gas comprising hydrocarbons and to utilize at least part of the second synthesis gas as heating medium in heat exchange within the gas heated steam methane reforming reactor, the gas heated steam methane reforming reactor being arranged for generating a third synthesis gas.

The gas heated steam methane reforming reactor is operable to utilize at least part of the second synthesis gas as heating media in heat exchange within the gas heated steam methane reforming reactor. The gas heated steam methane reforming reactor is arranged for generating a third synthesis gas over the third catalyst and for outputting the third synthesis gas from the reforming section as at least part of the output synthesis gas output from the reforming section. The overall heat efficiency of the chemical plant is increased by the addition of the gas heated steam methane reforming reactor, since the sensitive heat of the second synthesis gas is used within the gas heated steam methane reforming reactor. Moreover, when the chemical plant includes a gas heated steam methane reforming reactor, the overall output of the chemical plant is increased.

A gas heated steam methane reforming reactor is configured to use a hot gas to supply the heat for the endothermic steam methane reforming reaction by heat exchange, typically over a tube wall. An example of a configuration of a gas heated steam methane reforming reactor has several parallel tubes filled with catalyst which receive the feed gas. In the bottom of the reactor, the product gas from the catalyst filled tubes is mixed with part or all of the hot second synthesis gas from the autothermal reforming reactor to form a mixed gas. The mixed gas carries out heat exchange with the catalyst filled tubes within the gas heated methane reforming reactor and outputs the third synthesis gas. Other configurations of gas heated steam methane reforming reactors are also conceivable.

In an embodiment, the chemical plant further comprises:
- an post processing unit downstream the reforming section, where the post processing unit is arranged to receive the second and/or third synthesis gas and provide a post processed synthesis gas.

In an embodiment, the post processing unit is a post conversion unit having an inlet for allowing addition of gaseous stream comprising heated CO₂ to the second and/or third synthesis gas upstream the post conversion unit. The post processing unit houses a fourth catalyst active for catalyzing steam methane reforming, methanation and reverse water gas shift reactions. The post conversion unit is e.g. an adiabatic post conversion unit or a gas heat exchange reactor. The post processed synthesis gas is a synthesis gas with an H₂/CO ratio lower than the H₂/CO ratio of the output synthesis gas. By adding heated CO₂ and carrying out steam methane reforming, methanation and reverse water gas shift reactions in a separate reactor downstream the reforming section, the CO production of the process may be increased and/or the H₂/CO ratio may be tailored. The H₂/CO ratio of the post processed synthesis gas is e.g. lower than 1.8, lower than 1.5 or even lower than 1.0. The temperature of the heated CO₂ added may be e.g. a temperature of about 300°C, 400°C or even of about 500°C or above.

In an embodiment, the post processing unit is a water gas shift unit arranged to carry out the water gas shift reaction, thereby providing a post processed synthesis gas. In this embodiment, the post processed synthesis gas, is a water gas shifted synthesis gas, such as a hydrogen rich synthesis gas or a hydrogen gas stream. The water gas shift unit may be a single water gas shift unit, such as a medium temperature water gas shift unit, or a combination of two or more water gas shift units, e.g. a high temperature water gas shift unit and a low temperature water gas shift unit.

In an embodiment, the downstream section comprises gas separation unit(s) arranged to separate a stream of substantially pure CO₂, H₂, and/or CO from the output synthesis gas inlet to the downstream section, thereby providing a refined synthesis gas. Here, the term "substantially pure" is meant to denote the gas has at least 90% purity, such as 95% and preferably 99 % purity.

In an embodiment, the chemical plant further comprises a first separation unit arranged to separate the output synthesis gas or the post processed synthesis gas into a water condensate and an intermediate synthesis gas.

It is an advantage of the invention that the module M of the output synthesis gas and/or the intermediate synthesis gas may be tailored. The module M is the stoichiometric ratio (H₂-CO₂)/(CO+CO₂). The module M may be tailored to between 1.9 and 2.2, such as about 2.0 or 2.1, which is useful in the case where the downstream section comprises a methanol reactor arranged to convert the intermediate synthesis gas to methanol.

In an embodiment, the chemical plant further comprises a downstream section arranged to receive the intermediate synthesis gas and to process the intermediate synthesis gas to a chemical product and an off-gas. The chemical product is e.g. a hydrogen gas, a carbon monoxide gas, higher hydrocarbons, synthetic fuels, methanol, or ammonia.

In an embodiment, the chemical plant further comprises:
- a fired heater unit upstream the electrically heated reforming reactor, the fired heater unit being arranged to preheat the feed gas, and
- means for recycling at least part of the off-gas from the downstream section as fuel to the fired heater unit.

In order to ensure that the gas entering the autothermal reforming unit is sufficiently heated, a feed gas would typically be heated within a fired heater. In the chemical plant of the invention, the feed gas is heated and reformed within the electrically heated reforming reactor upstream the autothermal reforming reactor. Therefore, the heating within the fired heater unit may be reduced. In particular, the feed gas reaching the electrically heated reforming reactor may have a temperature of about 200-400°C and may exit the electrically heated reforming reactor as the first synthesis gas at between 450° and 850°C. When the fired heater is not required to heat the feed gas to more than about 400°, the fired heater may be reduced in size or even omitted.

When the reforming section comprises an electrically heated reforming reactor upstream the autothermal reforming reactor, the fired heater unit upstream the electrically heated reforming reactor may be reduced considerably in size. If the chemical plant comprises a prereformer upstream the reforming section, the fired heater unit is typically positioned upstream the prereformer. A fired heater may also be placed upstream a unit for purifying the feed gas (such as a desulfurization unit). In some cases one fired heater may be used for several pre-heating purposes by using two or more coils or coil sections.

By recycling off-gas from the downstream section back to the fired heater unit, it is rendered possible to maximize the use of hydrocarbons in the feed on the process side and minimize the direct use of such hydrocarbons of the fired heater unit. Thus, the overall consumption of hydrocarbons is minimized for a given output of combined synthesis gas from the reforming section. It is possible to balance the chemical plant so that the operation of the fired heater unit is adjusted to being primarily, or even fully, driven by heat supplied by burning a recycled off-gas. This allows for a minimum use of natural gas imported for being burned off for heat in the chemical plant, which in turn allows for an optimal utilization of feed gasses comprising hydrocarbons to the chemical plant. Typically, a relatively small amount of make-up gas comprising hydrocarbon is also fed to the fired heater unit in order to allow control of the duty of the fired heater unit. The term "duty" is in this context understood as the heat input added to or removed from a unit operation in a chemical plant. Moreover, the term "relative small amount" is meant to denote that less than 20 vol% of the fuel gas burned off in the fired heater unit is recycled off-gas. As an example, between 5 and 20 vol%, e.g. about 10 vol%, of the fuel gas burned off in the fired heater unit is recycled off-gas.

In an embodiment, the downstream section comprises gas separation unit(s) arranged to separate a stream of substantially pure CO₂, H₂, and/or CO from the intermediate synthesis gas, thereby providing a refined gas.

In an embodiment, the downstream section comprises an ammonia reactor to convert the intermediate synthesis gas or the refined synthesis gas to ammonia, a methanol reactor to convert the intermediate synthesis gas or the refined synthesis gas to methanol, or a Fischer-Tropsch reactor to convert the intermediate synthesis gas or the refined synthesis gas to a mixture of higher hydrocarbons.

Another aspect of the invention relates to a process for producing a chemical product from a feed gas comprising hydrocarbons, in a chemical plant comprising a reforming section. as from claim 13.

The reforming section comprises an electrically heated reforming reactor housing a first catalyst, and an autothermal reforming reactor downstream the electrically heated reforming reactor, where the autothermal reforming reactor houses a second catalyst. The process comprises the steps of:
- inletting the feed gas to the electrically heated reforming reactor and carrying out steam methane reforming to provide a first synthesis gas,
- inletting the first synthesis gas to the autothermal reforming reactor, and carrying out steam methane reforming to provide a second synthesis gas,
- outputting the output synthesis gas comprising the second synthesis gas from the reforming section.

It should be noted, that the term "carrying out reforming" in the autothermal reforming reactor is meant to denote the steam methane reforming taking place over the second catalyst. Other reactions, such as partial combustion of the hydrocarbon feed by sub-stoichiometric amounts of oxygen and water gas shift, take place in the autothermal reforming reactor.

Advantages of the process and embodiments thereof correspond to the advantages of the chemical plant and embodiments thereof and will therefore not be described in further detail here.

### SHORT DESCRIPTION OF THE FIGURES

Figure 1 shows a chemical plant according to an embodiment of the invention, where the reforming section comprises an electrically heated reforming reactor and an autothermal reactor in series;
Figure 2 shows a chemical plant according to an embodiment of the invention, where the reforming section comprises an electrically heated reforming reactor, an autothermal reactor and a gas heated steam methane reforming reactor;
Figures 3 and 4 show chemical plants according to embodiments of the invention, comprising a downstream section and recycling of an off-gas.

### DETAILED DESCRIPTION OF THE FIGURES

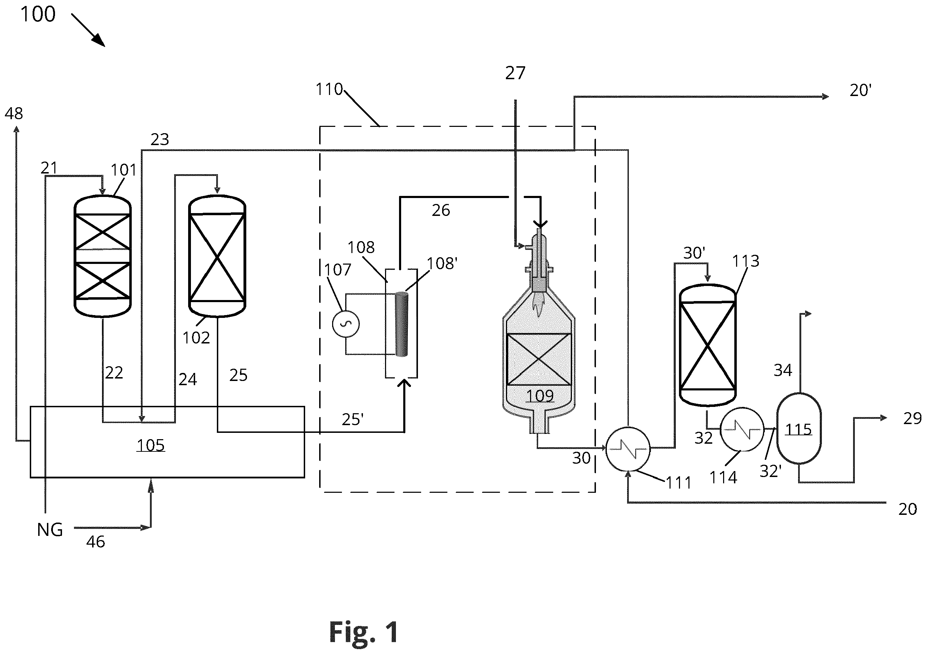

Figure 1 shows a chemical plant 100 according to an embodiment of the invention. The chemical plant 100 is a synthesis gas plant comprising a reforming section 110 with an electrically heated reforming reactor 108 housing a first catalyst and an autothermal reforming reactor 109 housing a second catalyst. The electrically heated reforming reactor 108 and autothermal reforming reactor 109 are arranged in series within the reforming section 110. The electrically heated reforming reactor 108 is arranged to receiving a feed gas 25', and to generate a first synthesis gas 26. The autothermal reforming reactor 109 is arranged to receive the first synthesis gas 26 as well as a stream 27 of oxidant gas. The stream 27 of oxidant gas comprises oxygen and may be e.g. air or oxygen, or a mixture of more than 90% oxygen with the balance being e.g nitrogen, steam and/or argon.

During operation of the chemical plant 100, a feed gas 21 comprising hydrocarbons undergoes feed purification in a desulfurization unit 101 and becomes a desulfurized feed gas 22. The feed gas 21 comprising hydrocarbons is e.g. natural gas or town gas. The desulfurized feed gas 22 is preheated in a fired heater unit 105 and steam 23 is added, resulting in a gas stream 24. The gas stream 24 is led to a prereforming unit 102 housing steam reforming catalyst. Typically, the prereforming unit 102 is an adiabatic prereforming unit, wherein higher hydrocarbons are reacted so that the prereformed feed gas 25 exiting the prereformer contains no or very small amounts of higher hydrocarbons. The prereformed feed gas 25 is heated in the fired heater unit 105 to a heated prereformed feed gas 25', which is led to the electrically heated reforming reactor 108.

The heated prereformed feed gas 25' undergoes steam methane reforming in the electrically heated reforming reactor 108, and a first synthesis gas 26 is output from the electrically heated reforming reactor 108. The first synthesis gas 26 is input to the autothermal reforming reactor 109, wherein it undergoes partial combustion together with by sub-stoichiometric amounts of oxygen from the stream 27, followed by steam reforming of the partially combusted hydrocarbon feed gas in a fixed bed of the second catalyst. The second catalyst is a steam methane reforming catalyst. A resulting second synthesis gas 30 is output from the autothermal reforming reactor 109. The second synthesis gas 30 is output from the reforming section as the output synthesis gas.

The electrically heated reforming reactor 108 e.g. comprises a pressure shell housing an electrical heating unit 108' arranged to heat the first catalyst. The first catalyst is operable to catalyzing steam reforming of the feed gas. The pressure shell has a design pressure of between 5 and 45 bar. A heat insulation layer may be adjacent to at least part of the inside of said pressure shell. At least two conductors are electrically connected to the electrical heating unit and to an electrical power supply 107 is placed outside the pressure shell. The electrical power supply 107 is dimensioned to heat at least part of the first catalyst to a temperature of at least 500°C by passing an electrical current through the electrical heating unit.

The output synthesis gas 30 is cooled in a heat exchanger 111 to a cooled synthesis gas 30'. The cooled synthesis gas 30' enters a post processing unit 113, e.g. a water gas shift unit, and a water gas shifted synthesis gas 32 exits the water gas shift unit 113. The water gas shifted synthesis gas 32 is cooled in a second heat exchanger 114 to a cooled water gas shifted synthesis gas 32', which enters the first separation unit 115. The first separation unit 115 e.g. comprises a flash separation unit. The cooled water gas shifted synthesis gas 32' thus enters the flash separation unit 115 arranged to separate the cooled water gas shifted synthesis gas 32' into water 29 and an intermediate synthesis gas 34, viz. a dry synthesis gas. Optionally, the intermediate synthesis gas 34 may enter a PSA unit (not shown in figure 1) arranged to separate the intermediate synthesis gas 34 into a product synthesis gas in the form of a stream of substantially pure hydrogen and an off-gas. A heat exchange fluid 20, such as water, is used for heat exchange in the heat exchanger 111 and a heated heat exchange fluid, such as steam, is exported as stream 20'.

It should be noted, that the chemical plant 100 typically comprises further equipment, such as compressors, heat exchangers etc.; however, such further equipment is not shown in figure 1. Moreover, it should be noted that even though figure 1 shows a purification unit in the form of a desulfurization unit 101 and a prereforming unit 102, such units need not be part of the chemical plant 100.

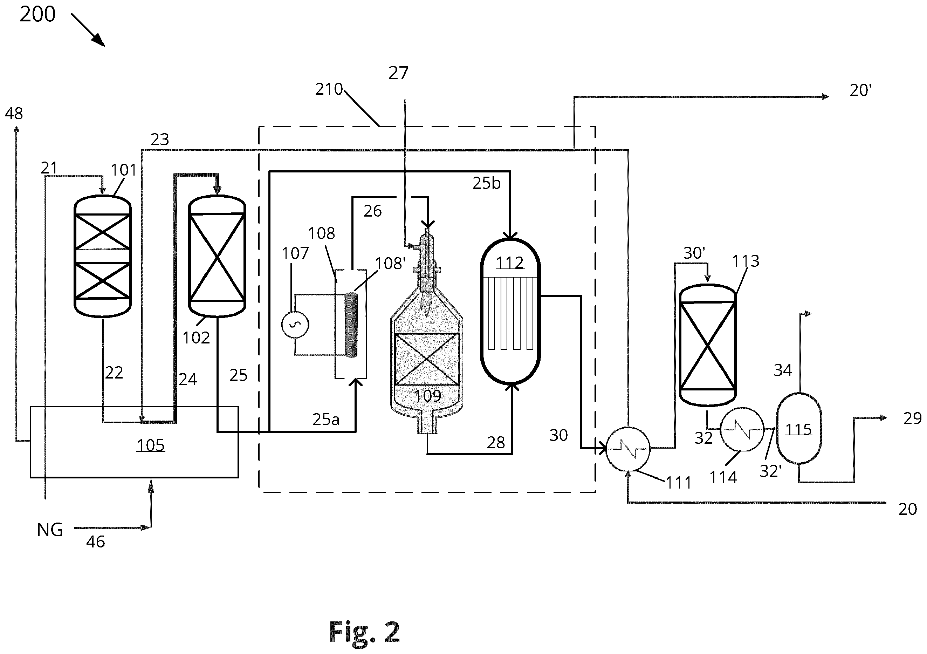

Figure 2 shows a chemical plant 200 according to an embodiment of the invention. The chemical plant 200 is a synthesis gas plant comprising a reforming section 210 with an electrically heated reforming reactor 108 housing a first catalyst, an autothermal reforming reactor 109 housing a second catalyst and a gas heated steam methane reforming reactor 112 housing a third catalyst. The electrically heated reforming reactor 108 and autothermal reforming reactor 109 are arranged in series within the reforming section 210. The electrically heated reforming reactor 108 is arranged to receive a feed gas 25', and to generate a first synthesis gas 26. The autothermal reforming reactor 109 is arranged to receive the first synthesis gas 26 as well as a stream 27 of oxidant gas and to generate a second synthesis gas 28. The stream 27 of oxidant gas comprises oxygen and may be e.g. air or oxygen, or a mixture of more than 90% oxygen with the balance being e.g nitrogen, steam, and/or argon.

During operation of the chemical plant 100, a feed gas 21 comprising hydrocarbons undergoes feed purification in a desulfurization unit 101 and becomes a desulfurized feed gas 22. The feed gas 21 comprising hydrocarbons is e.g. natural gas or town gas. The desulfurized feed gas 22 is preheated in a fired heater unit 105 and steam 23 is added, resulting in a gas stream 24. The gas stream 24 is led to a prereforming unit 102 housing steam reforming catalyst. Typically, the prereforming unit 102 is an adiabatic prereforming unit, wherein higher hydrocarbons are reacted so that the prereformed feed gas 25 exiting the prereformer contains no or very small amounts of higher hydrocarbons. The prereformed feed gas 25 is heated in the fired heater unit 105 to a heated prereformed feed gas 25', which is led to the electrically heated reforming reactor 108.

A fuel gas 46 comprising hydrocarbons, e.g. natural gas, to is sent to the fired heater unit in order to be burned off to provide heat within the fired heater unit 105. An effluent gas 48 is output from the fired heater unit 105.

A first part 25a of the heated prereformed feed gas 25' undergoes steam methane reforming in the electrically heated reforming reactor 108, and a first synthesis gas 26 is output from the electrically heated reforming reactor 108. The first synthesis gas 26 is input to the autothermal reforming reactor 109, wherein it undergoes partial combustion together with by sub-stoichiometric amounts of oxygen from the stream 27, followed by steam reforming of the partially combusted hydrocarbon feed gas in a fixed bed of the second catalyst. The second catalyst is a steam reforming catalyst. A resulting second synthesis gas 30 is output from the autothermal reforming reactor 109.

The second synthesis gas 28 is inlet to the gas heated steam methane reforming reactor 112 in order to provide heat for the steam methane reforming reaction of the second part 25b of the feed gas 25 entering the gas heated steam methane reforming reactor 112 from another side. The gas exiting the gas heated steam methane reforming reactor 112 is a third synthesis gas 30. The third synthesis gas 30 is the output synthesis gas output from the reforming section.

The output synthesis gas 30 is cooled in a heat exchanger 111 to a cooled synthesis gas 30'. The cooled synthesis gas 30' enters a post processing unit 113, e.g. a water gas shift unit, and a water gas shifted synthesis gas 32 exits the water gas shift unit 113. The water gas shifted synthesis gas 32 is cooled in a second heat exchanger 114 to a cooled water gas shifted synthesis gas 32', which enters the first separation unit 115. The first separation unit 115 e.g. comprises a flash separation unit. The cooled water gas shifted synthesis gas 32' thus enters the flash separation unit 115 arranged to separate the cooled water gas shifted synthesis gas 32' into water 29 and an intermediate synthesis gas 34, viz. a dry synthesis gas. Optionally, the intermediate synthesis gas 34 may enter a PSA unit (not shown in figure 1) arranged to separate the intermediate synthesis gas 34 into a product synthesis gas in the form of a stream of substantially pure hydrogen and an off-gas . A heat exchange fluid 20, such as water, is used for heat exchange in the heat exchanger 111 and a heated heat exchange fluid, such as steam, is exported as stream 20'.

It should be noted, that the chemical plant 100 typically comprises further equipment, such as compressors, heat exchangers etc.; however, such further equipment is not shown in figure 1. Moreover, it should be noted that even though figure 1 shows a purification unit in the form of a desulfurization unit 101 and a prereforming unit 102, such units need not be part of the chemical plant 100.

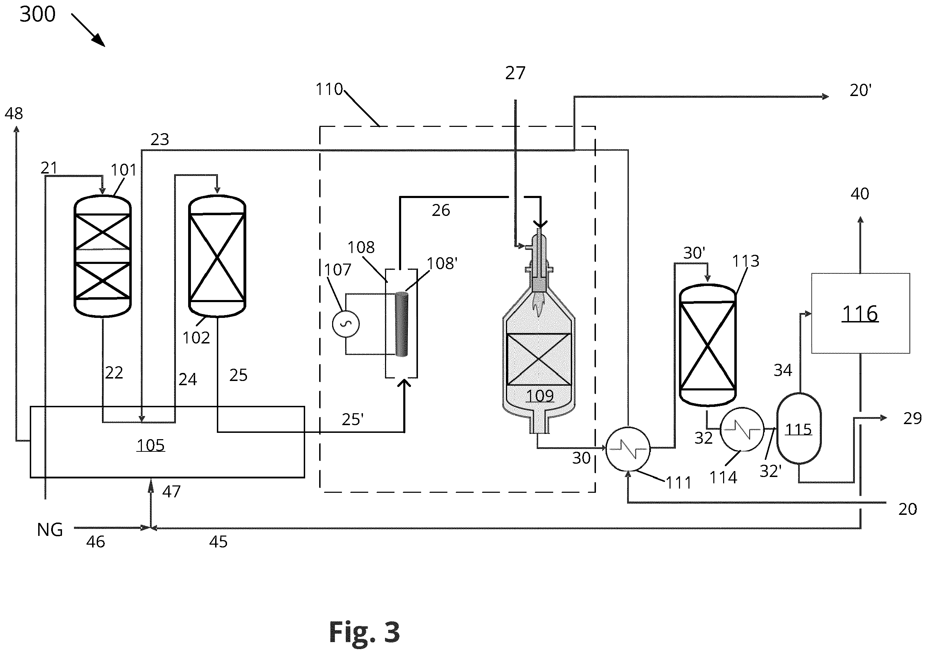

Figure 3 shows a chemical plant according to embodiments of the invention, comprising a downstream section and recycling of an off-gas. Thus, figure 3 includes the units of the chemical plant shown in figure 1 in addition to further units. The parts that are the same for figure 1 and 3 will not be described in detail below.

The chemical plant 300 of figure 3 thus includes the desulfurization unit 101, the prereforming unit 102, the fired heater unit 105, the reforming section 110, the post processing unit 113, the first separation unit 115, and the heat exchangers 111 and 114 as described in relation to figure 1.

The output synthesis gas 30 exiting from the reforming section 110 is cooled in the heat exchanger 111 to a cooled synthesis gas 30'. The cooled synthesis gas 30' enters the post processing unit 113, here in the form of a water gas shift unit, and a water gas shifted synthesis gas 32 exits the water gas shift unit 113. The water gas shifted synthesis gas 32 is cooled in a second heat exchanger 114 to a cooled water gas shifted synthesis gas 32', which enters the first separation unit 115, such as e.g. a flash separation unit 115 arranged to separate the cooled water gas shifted synthesis gas 32' into a condensate 29 and an intermediate synthesis gas 34, viz. a dry synthesis gas. The intermediate synthesis gas 34 enters the downstream section 116 arranged to process the intermediate synthesis gas 34 to a chemical product 40 and an off-gas 45. The downstream section 116 comprises e.g. an ammonia reactor to convert the intermediate synthesis gas 34 to ammonia, a methanol reactor to convert the intermediate synthesis gas 34 gas to methanol, or a Fischer-Tropsch reactor to convert the intermediate synthesis gas 34 to a mixture of higher hydrocarbons.

The off-gas 45 from the downstream section 116 is recycled as fuel to one or more burners of the fired heater unit 105. The off-gas 45 is combined with a small amount of make-up gas 46 comprising hydrocarbons, e.g. natural gas, to form the fuel gas 47 sent to the one or more burners of the fired heater unit 105. The fired heater unit 105 is arranged to provide heat for preheating the feed gas 21, the desulfurized feed gas 22, and the prereformed feed gas 25. An effluent gas 48 is output from the fired heater unit 105.

A fuel gas 46 comprising hydrocarbons, e.g. natural gas, to is sent to the fired heater unit in order to be burned off to provide heat within the fired heater unit 105. An effluent gas 48 is output from the fired heater unit 105.

A heat exchange fluid 20, such as water, is used for heat exchange in the heat exchanger 111 and a heated heat exchange fluid, such as steam, is exported as stream 20'. A part of the steam is used as addition of steam 23 to the desulfurized feed gas 22.

By recycling off-gas from the downstream section 116 back to the fired heater unit 105, it is rendered possible to maximize the use of hydrocarbons in the feed on the process side and minimize the use of natural gas within the fired heater unit 105. It is possible to balance the chemical plant so that the operation of the fired heater unit 105 is adjusted to being primarily, or even fully, driven by heat supplied by burning a recycled off-gas 45. This allows for a minimum use of natural gas imported for being burned off for heat in the chemical plant 300, which in turn allows for an optimal utilization of feed gasses comprising hydrocarbons to the chemical plant. Typically, a relatively small amount of make-up gas 46 comprising hydrocarbons, e.g. natural gas, is also fed to the fired heater unit 105 in order to allow for control of the duty of the fired heater unit. The term "duty" is in this context understood as the heat input added to or removed from a unit operation in a chemical plant.

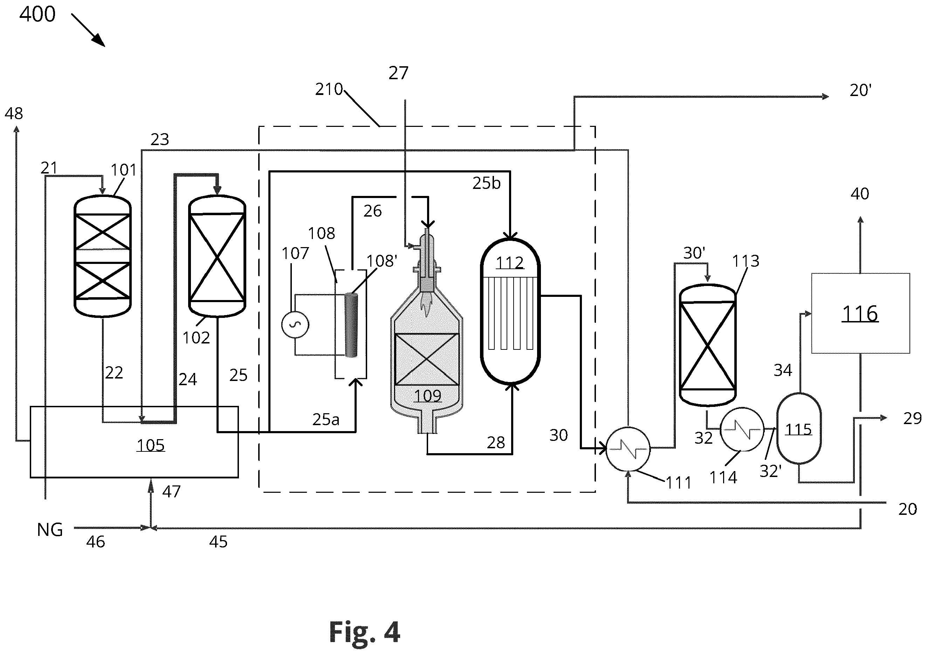

Figure 4 shows a chemical plant 400 according to an embodiment of the invention, comprising a downstream section and recycling of an off-gas. Figure 4 includes the units of the chemical plants shown in figure 2, in addition to further units. The parts that are the same for figure 2 and 4 will not be described in detail below.

The chemical plant 300 of figure 3 thus includes the desulfurization unit 101, the prereforming unit 102, the fired heater unit 105, the reforming section 210, the post processing unit 113, the first separation unit 115, and the heat exchangers 111 and 114 as described in relation to figure 2.

The output synthesis gas 30 exiting from the reforming section 210 is cooled in the heat exchanger 111 to a cooled synthesis gas 30'. The cooled synthesis gas 30' enters the post processing unit 113, here in the form of a water gas shift unit, and a water gas shifted synthesis gas 32 exits the water gas shift unit 113. The water gas shifted synthesis gas 32 is cooled in a second heat exchanger 114 to a cooled water gas shifted synthesis gas 32', which enters the first separation unit 115, such as e.g. a flash separation unit 115 arranged to separate the cooled water gas shifted synthesis gas 32' into a condensate 29 and an intermediate synthesis gas 34, viz. a dry synthesis gas. The intermediate synthesis gas 34 enters the downstream section 116 arranged to process the intermediate synthesis gas 34 to a chemical product 40 and an off-gas 45. The downstream section 116 comprises e.g. an ammonia reactor to convert the intermediate synthesis gas 34 to ammonia, a methanol reactor to convert the intermediate synthesis gas 34 gas to methanol, or a Fischer-Tropsch reactor to convert the intermediate synthesis gas 34 to a mixture of higher hydrocarbons.

The off-gas 45 from the downstream section 116 is recycled as fuel to one or more burners of the fired heater unit 105. The off-gas 45 is combined with a small amount of make-up gas 46 comprising hydrocarbons, e.g. natural gas, to form the fuel gas 47 sent to the one or more burners of the fired heater unit 105. The fired heater unit 105 is arranged to provide heat for preheating the feed gas 21, the desulfurized feed gas 22, and the prereformed feed gas 25.

A heat exchange fluid 20, such as water, is used for heat exchange in the heat exchanger 111 and a heated heat exchange fluid, such as steam, is exported as stream 20'. A part of the steam is used as addition of steam 23 to the desulfurized feed gas 22.

By recycling off-gas from the downstream section 116 back to the fired heater unit 105, it is rendered possible to maximize the use of hydrocarbons in the feed on the process side and minimize the use of natural gas within the fired heater unit 105. It is possible to balance the chemical plant so that the operation of the fired heater unit 105 is adjusted to being primarily, or even fully, driven by heat supplied by burning a recycled off-gas 45. This allows for a minimum use of natural gas imported for being burned off for heat in the chemical plant 300, which in turn allows for an optimal utilization of feed gasses comprising hydrocarbons to the chemical plant. Typically, a relatively small amount of make-up gas 46 comprising hydrocarbons, e.g. natural gas, is also fed to the fired heater unit 105 in order to allow for control of the duty of the fired heater unit 105. The term "duty" is in this context understood as the heat input added to or removed from a unit operation in a chemical plant.

The embodiments shown in figures 1 to 4 all show a feed gas entering into the electrically heated reforming reactor from the lower side of the electrically heated reforming reactor. It is understood that this is not necessarily the case and that the feed gas may enter the electrically heated reforming reactor from the top or the side, if appropriate.

It should be noted, that the chemical plants shown in figures 1 to 4 typically comprise further equipment, such as compressors, heat exchangers etc.; however, such further equipment is not shown in the figures.

### EXAMPLE:

In the classical 2-step reforming methanol layout (2-step-MeOH), the chemical plant comprises a reforming section with a primary reformer in the form of a steam methane reformer (SMR) and a methanol section. Natural gas is used as the principal reformer feed and is prereformed and fed to the primary reformer. Heating of the SMR is provided by natural gas in some part and expanded off-gas from the methanol (MeOH) synthesis which comes in part from the MeOH loop and the MeOH distillation. The partially reformed gas from the primary reformer is sent to a secondary reformer which is supplied with oxygen, typically from an air separation unit (ASU), where oxygen addition is controlled in order to obtain the desired module of the synthesis gas. The synthesis gas from the reforming section is cooled to remove water in the process gas, before it is compressed to 92 barg. The compressor was driven by a steam turbine, operated on superheated steam produced in the waste-heat section of the SMR. The compressed synthesis gas was combined with recycle gas from the methanol loop and sent to the methanol reactor of the methanol section and the resulting product was refined to a final pure methanol product.

In this example, a 2-step reforming methanol layout (2-step-MeOH) as described above is compared with an electrically heated reformer (2-step eSMR-MeOH). Making a 2-step reforming layout with an electrically heated reformer (eSMR) was done by also including a fired heater for feed preheating; the fired heater was fueled partly by expanded off-gas from the methanol section and natural gas. In this specific case, additional burning of natural gas was required to provide sufficient superheated steam to drive the turbine(s).

Comparing consumption figures of the two layouts is shown in Table 1. Using an electrically heated reformer (eSMR) in the 2-step reforming layout in 2-step-eSMR-MeOH was found to have the same feed consumption as the classical 2-step-MeOH layout. However, a large difference is found on the fuel side, where the majority of the natural gas consumption was replaced with electricity. This, obviously, translate into lower CO₂ emissions from the 2-step-eSMR-MeOH layout, which is associated with the reduced flue gas emissions.

**Table 1**

| | 2-step-MeOH | 2-step-eSMR-MeOH |
|---|---|---|
| **Consumption:** | | |
| Natural gas feed [kNm³/h] | 146.6 | 146.6 |
| Natural gas fuel [Nm³/h] | 20.6 | 2.7 |
| Electricity work [MW] | 39.2 | 213.9 |

| **Production:** | | |
|---|---|---|
| MeOH product [MTPD] | 5000 | 5000 |
| CO₂ emissions (plant) [kNm³/h] | 28.6 | 11.6 |

## Claims

1. A chemical plant comprising:
- a reforming section arranged to receive a feed gas comprising hydrocarbons including methane and provide an output synthesis gas, wherein said reforming section comprises:
- an electrically heated reforming reactor housing a first catalyst, said electrically heated reforming reactor being arranged for receiving said feed gas and generating a first synthesis gas by a steam methane reaction,
- an autothermal reforming reactor downstream said electrically heated reforming reactor, said autothermal reforming reactor housing a second catalyst, said autothermal reforming reactor being arranged for receiving said first synthesis gas and outputting a second synthesis gas, wherein said reforming section is arranged to output said output synthesis gas comprising said second synthesis gas,
wherein said electrically heated reforming reactor comprises:
- a pressure shell housing an electrical heating unit arranged to heat said first catalyst, where said first catalyst is operable to catalyzing steam reforming of said feed gas,
wherein said pressure shell has a design pressure of between 5 and 90 bar,
- a heat insulation layer adjacent to at least part of the inside of said pressure shell, and
- at least two conductors electrically connected to said electrical heating unit and to an electrical power supply placed outside said pressure shell,
wherein said electrical power supply is dimensioned to heat at least part of said first catalyst to a temperature of at least 450°C by passing an electrical current through said electrical heating unit,
wherein said electrical heating unit comprises a macroscopic structure of electrically conductive material, where said macroscopic structure supports a ceramic coating and said ceramic coating supports said first catalyst.

2. The chemical plant according to claim 1, further comprising a prereformer upstream said electrically heated reforming reactor.

3. The chemical plant according to any of the claims 1 to 2, wherein said reforming section furthermore comprises a gas heated steam methane reforming reactor in parallel to said electrically heated reforming reactor and said autothermal reforming reactor, wherein said gas heated steam methane reforming reactor comprises a third catalyst and being operable to receive a second feed gas comprising hydrocarbons and to utilize at least part of said second synthesis gas as heating medium in heat exchange within said gas heated steam methane reforming reactor, said gas heated steam methane reforming reactor being arranged for generating a third synthesis gas.

4. The chemical plant according to any of the claims 1 to 3, further comprising:
- a post processing unit downstream the reforming section, where said post processing unit is arranged to receive said output synthesis gas and provide a post processed synthesis gas.

5. The chemical plant according to claim 4, wherein said post processing unit is a post conversion unit having an inlet for allowing addition of heated CO₂ to said output synthesis gas upstream the post conversion unit and housing a fourth catalyst active for catalyzing steam methane reforming, methanation and reverse water gas shift.

6. The chemical plant according to claim 4 or 5, wherein said post processing unit is a water gas shift unit arranged to carry out the water gas shift reaction, thereby providing a post processed synthesis gas.

7. The chemical plant according to any of the claims 1 to 6, further comprising:
- a first separation unit arranged to separate said output synthesis gas or said post processed synthesis gas into a water condensate and an intermediate synthesis gas.

8. The chemical plant according to any of the claim 7, further comprising:
- a downstream section arranged to receive the intermediate synthesis gas and to process the intermediate synthesis gas to a chemical product and an off-gas.

9. The chemical plant according to claim 8, further comprising:
- a fired heater unit upstream said electrically heated reforming reactor, the fired heater unit being arranged to preheat the said feed gas, and
- means for recycling at least part of said off-gas from said downstream section as fuel to the fired heater unit.

10. The chemical plant according to any of the claims 8-9, wherein said downstream section comprises gas separation unit(s) arranged to separate a stream of substantially pure CO₂, H₂, and/or CO from said intermediate synthesis gas, thereby providing a refined synthesis gas.

11. The chemical plant according to any of the claims 8 to 10, wherein said downstream section comprises an ammonia reactor to convert said intermediate synthesis gas or said refined synthesis gas to ammonia, a methanol reactor to convert said intermediate synthesis gas or said refined synthesis gas to methanol, or a Fischer-Tropsch reactor to convert said intermediate synthesis gas or said refined synthesis gas to a mixture of higher hydrocarbons.

12. The chemical plant according to any of claims 1-11, wherein the electrical power supply and the electrical heating unit within the pressure shell are dimensioned so that at least part of the electrical heating unit reaches a temperature of 450°C-850°C, such as 500°C -750°C, and preferably 550°C -650°C.

13. A process for producing a chemical product from a feed gas comprising hydrocarbons including methane, in a chemical plant comprising a reforming section, said reforming section comprising an electrically heated reforming reactor housing a first catalyst, and an autothermal reforming reactor downstream said electrically heated reforming reactor, said autothermal reforming reactor housing a second catalyst, said process comprising the steps of:
- inletting said feed gas to said electrically heated reforming reactor and carrying out steam methane reforming to provide a first synthesis gas,
- inletting said first synthesis gas to said autothermal reforming reactor, and carrying out steam methane reforming to provide a second synthesis gas,
- outputting a synthesis gas comprising said second synthesis gas from said reforming section,
wherein said electrically heated reforming reactor comprises a pressure shell housing an electrical heating unit arranged to heat said first catalyst, wherein said first catalyst is operable to catalyze steam reforming of said feed gas, wherein said pressure shell has a design pressure of between 5 and 90 bar,
- a heat insulation layer adjacent to at least part of the inside of said pressure shell, and
- at least two conductors electrically connected to said electrical heating unit and to an electrical power supply placed outside said pressure shell,
wherein said process further comprises the steps of:
- Pressurizing said feed gas to a pressure of between 5 and 90 bar upstream said electrically heated reforming reactor,
- passing an electrical current through said electrical heating unit thereby heating at least part of said first catalyst to a temperature of at least 450°C,
wherein said electrical heating unit comprises a macroscopic structure of electrically conductive material, where said macroscopic structure supports a ceramic coating and said ceramic coating supports said first catalyst.

14. The process according to claim 13, further comprising the step of adding one or more additional feed streams to the reforming section upstream the electrically heated reformer, to the first synthesis gas, and/or directly to the autothermal reforming reactor.

15. The process according to claim 14, wherein a tail gas from a Fischer-Tropsch unit is added to the first synthesis gas or directly to the autothermal reforming reactor.

16. The process according to any of the claims 13 to 15, further comprising the step of prereforming said feed gas in a prereformer upstream said electrically heated reforming reactor.

17. The process according to any of the claims 13 to 16, wherein said reforming section furthermore comprises a gas heated steam methane reforming reactor in parallel to said electrically heated reforming reactor and said autothermal reforming reactor, wherein said gas heated steam methane reforming reactor comprises a third catalyst, said process furthermore comprising the steps of:
- inletting a second feed comprising hydrocarbons into said gas heated steam methane reforming reactor,
- utilizing at least part of said second synthesis gas as heating media in heat exchange within said gas heated steam methane reforming reactor,
- generating a third synthesis gas over the third catalyst within the gas heated steam methane reforming reactor, and
- outputting said third synthesis gas from said reforming section as at least part of said output synthesis gas.

18. The process according to any of the claims 13 to 17, further comprising:
- in a post processing unit downstream said reforming section, post processing said output synthesis gas to provide a post processed synthesis gas.

19. The process according to claim 18, wherein said post processing unit is a post conversion unit housing a fourth catalyst active for catalyzing steam methane reforming, methanation and reverse water gas shift reactions, wherein said process furthermore comprises the step of inletting heated CO₂ to said output synthesis gas upstream said post conversion unit.

20. The process according to claim 18 or 19, wherein said post processing unit is a water gas shift unit and the step of post processing said output synthesis gas comprises carrying out the water gas shift reaction.

21. The process according to any of the claims 13 to 20, further comprising the step of:
- separating said output synthesis gas or said post processed synthesis gas into a water condensate and an intermediate synthesis gas in a first separation unit downstream said post processing unit.

22. The process according to claim 21, further comprising the step of:
- providing said intermediate synthesis gas to a downstream section arranged to receive the intermediate synthesis gas and to process the intermediate synthesis gas to said chemical product and an off-gas.

23. The process according to claim 22, further comprising:
- providing fuel to a fired heater unit upstream said autothermal reforming reactor, said fired heater unit being operable to preheat said feed gas, and
- recycling at least part of said off-gas from said downstream section as fuel to the fired heater unit.

24. The process according to any of the claims 21 to 23, wherein said process comprises separating a stream of substantially pure CO₂, H₂, and/or CO from said intermediate synthesis gas, thereby providing a refined synthesis gas, in one or more gas separation unit(s) of said downstream section.

25. The process according to any of the claims 21 to 24, wherein said process further comprises: converting said intermediate synthesis gas to ammonia in an ammonia reactor of said downstream section, to convert said intermediate synthesis gas to methanol in a methanol reactor of said downstream section, or to convert said intermediate synthesis gas to a mixture of higher hydrocarbons in a Fischer-Tropsch reactor.

## Patentansprüche

1. Chemische Anlage, umfassend:
- einen Reformierungsabschnitt, der eingerichtet ist zur Aufnahme eines Einsatzgases, das Kohlenwasserstoffe einschließlich Methan umfasst, und zur Bereitstellung eines Ausgabesynthesegases, wobei der Reformierungsabschnitt umfasst:
- einen elektrisch beheizten Reformierungsreaktor, in dem ein erster Katalysator untergebracht ist, wobei der elektrisch beheizte Reformierungsreaktor zur Aufnahme des Einsatzgases und zur Erzeugung eines ersten Synthesegases durch eine Dampf-Methan-Reaktion eingerichtet ist,
- einen autothermen Reformierungsreaktor stromabwärts des elektrisch beheizten Reformierungsreaktors, wobei in dem autothermen Reformierungsreaktor ein zweiter Katalysator untergebracht ist, wobei der autotherme Reformierungsreaktor zur Aufnahme des ersten Synthesegases und zur Ausgabe eines zweiten Synthesegases eingerichtet ist, wobei der Reformierungsabschnitt zur Ausgabe des Ausgabesynthesegases, umfassend das zweite Synthesegas, eingerichtet ist,
wobei der elektrisch beheizte Reformierungsreaktor umfasst:
- einen Druckmantel, in dem eine elektrische Heizeinheit untergebracht ist, die zur Erwärmung des ersten Katalysators eingerichtet ist, wobei der erste Katalysator so betrieben werden kann, dass er eine Dampfreformierung des Einsatzgases katalysiert, wobei der Druckmantel einen Auslegungsdruck zwischen 5 und 90 bar aufweist,
- eine Wärmeisolationsschicht, die zumindest an einen Teil des Inneren des Druckmantels angrenzt, und
- mindestens zwei Leiter, die elektrisch mit der elektrischen Heizeinheit und mit einer außerhalb des Druckmantels angeordneten elektrischen Energieversorgung verbunden sind,
wobei die elektrische Energieversorgung so ausgelegt ist, dass sie mindestens einen Teil des ersten Katalysators auf eine Temperatur von mindestens 450°C erhitzt, indem sie einen elektrischen Strom durch die elektrische Heizeinheit leitet, wobei die elektrische Heizeinheit eine makroskopische Struktur aus elektrisch leitfähigem Material umfasst, wobei die makroskopische Struktur eine Keramikbeschichtung trägt und die Keramikbeschichtung den ersten Katalysator trägt.

2. Chemische Anlage nach Anspruch 1, die ferner einen Vorreformer stromaufwärts des elektrisch beheizten Reformierungsreaktors umfasst.

3. Chemische Anlage nach einem der Ansprüche 1 bis 2, wobei der Reformierungsabschnitt zudem einen gasbeheizten Dampf-Methan-Reformierungsreaktor parallel zu dem elektrisch beheizten Reformierungsreaktor und dem autothermen Reformierungsreaktor umfasst, wobei der gasbeheizte Dampf-Methan-Reformierungsreaktor einen dritten Katalysator umfasst und so betrieben werden kann, dass er ein zweites, Kohlenwasserstoffe umfassendes Einsatzgas aufnimmt und zumindest einen Teil des zweiten Synthesegases als Heizmedium im Wärmeaustausch innerhalb des gasbeheizten Dampf-Methan-Reformierungsreaktors nutzt, wobei der gasbeheizte Dampf-Methan-Reformierungsreaktor zur Erzeugung eines dritten Synthesegases eingerichtet ist.

4. Chemische Anlage nach einem der Ansprüche 1 bis 3, ferner umfassend:
- eine Nachbearbeitungseinheit stromabwärts des Reformierungsabschnitts, wobei die Nachbearbeitungseinheit zur Aufnahme des Ausgabesynthesegases und zur Bereitstellung eines nachbearbeiteten Synthesegases eingerichtet ist.

5. Chemische Anlage nach Anspruch 4, wobei die Nachbearbeitungseinheit eine Nachkonvertierungseinheit ist, die einen Einlass aufweist, der die Zugabe von erhitztem CO₂ zu dem Ausgabesynthesegas stromaufwärts der Nachkonvertierungseinheit ermöglicht, und in der ein vierter Katalysator untergebracht ist, der aktiv ist für das Katalysieren von Dampf-Methan-Reformierung, Methanisierung und reversem Wassergas-Shift.

6. Chemische Anlage nach Anspruch 4 oder 5, wobei die Nachbearbeitungseinheit eine Wassergas-Shift-Einheit ist, die zur Durchführung der Wassergas-Shift-Reaktion eingerichtet ist, wodurch ein nachbearbeitetes Synthesegas bereitgestellt wird.

7. Chemische Anlage nach einem der Ansprüche 1 bis 6, ferner umfassend:
- eine erste Trenneinheit, die zur Trennung des Ausgabesynthesegases oder des nachbearbeiteten Synthesegases in ein Wasserkondensat und ein intermediäres Synthesegas eingerichtet ist.

8. Chemische Anlage nach Anspruch 7, ferner umfassend:
- einen stromabwärts gelegenen Abschnitt, der zur Aufnahme des intermediären Synthesegases und zur Verarbeitung des intermediären Synthesegases zu einem chemischen Produkt und einem Abgas eingerichtet ist.

9. Chemische Anlage nach Anspruch 8, ferner umfassend:
- eine befeuerte Heizeinheit stromaufwärts des elektrisch beheizten Reformierungsreaktors, wobei die befeuerte Heizeinheit zum Vorwärmen des Einsatzgases eingerichtet ist, und
- Mittel zur Rückführung mindestens eines Teils des Abgases aus dem stromabwärts gelegenen Abschnitt als Brennstoff in die befeuerte Heizeinheit.

10. Chemische Anlage nach einem der Ansprüche 8 bis 9, wobei der stromabwärts gelegene Abschnitt eine oder mehrere Gastrenneinheiten umfasst, die zur Abtrennung eines Stroms aus im Wesentlichen reinem CO₂, H₂ und/oder CO von dem intermediären Synthesegas eingerichtet ist(sind), wodurch ein raffiniertes Synthesegas bereitgestellt wird.

11. Chemische Anlage nach einem der Ansprüche 8 bis 10, wobei der stromabwärts gelegene Abschnitt einen Ammoniakreaktor zur Umwandlung des intermediären Synthesegases oder des raffinierten Synthesegases in Ammoniak, einen Methanolreaktor zur Umwandlung des intermediären Synthesegases oder des raffinierten Synthesegases in Methanol oder einen Fischer-Tropsch-Reaktor zur Umwandlung des intermediären Synthesegases oder des raffinierten Synthesegases in ein Gemisch aus höheren Kohlenwasserstoffen umfasst.

12. Chemische Anlage nach einem der Ansprüche 1 bis 11, wobei die elektrische Energieversorgung und die elektrische Heizeinheit innerhalb des Druckmantels so dimensioniert sind, dass zumindest ein Teil der elektrischen Heizeinheit eine Temperatur von 450°C bis 850°C, wie 500°C bis 750°C, und vorzugsweise 550°C bis 650°C erreicht.

13. Verfahren zur Herstellung eines chemischen Produkts aus einem Einsatzgas, umfassend Kohlenwasserstoffe einschließlich Methan, in einer chemischen Anlage, umfassend einen Reformierungsabschnitt, wobei der Reformierungsabschnitt einen elektrisch beheizten Reformierungsreaktor, in dem ein erster Katalysator untergebracht ist, und einen autothermen Reformierungsreaktor stromabwärts des elektrisch beheizten Reformierungsreaktors umfasst, wobei in dem autothermen Reformierungsreaktor ein zweiter Katalysator untergebracht ist, wobei das Verfahren die folgenden Schritte umfasst:
- Einführen des Einsatzgases in den elektrisch beheizten Reformierungsreaktor und Durchführen einer Dampf-Methan-Reformierung, um ein erstes Synthesegas bereitzustellen,
- Einführen des ersten Synthesegases in den autothermen Reformierungsreaktor und Durchführen einer Dampf-Methan-Reformierung, um ein zweites Synthesegas bereitzustellen,
- Ausgeben eines Synthesegases, umfassend das zweite Synthesegas, aus dem Reformierungsabschnitt,
wobei der elektrisch beheizte Reformierungsreaktor einen Druckmantel umfasst, in dem eine elektrische Heizeinheit untergebracht ist, die zur Erwärmung des ersten Katalysators eingerichtet ist, wobei der erste Katalysator so betrieben werden kann, dass er die Dampfreformierung des Einsatzgases katalysiert, wobei der Druckmantel einen Auslegungsdruck zwischen 5 und 90 bar aufweist,
- eine Wärmeisolationsschicht, die zumindest an einen Teil des Inneren des Druckmantels angrenzt, und
- mindestens zwei Leiter, die elektrisch mit der elektrischen Heizeinheit und mit einer außerhalb des Druckmantels angeordneten elektrischen Energieversorgung verbunden sind,
wobei das Verfahren ferner die folgenden Schritte umfasst:
- Druckbeaufschlagung des Einsatzgases auf einen Druck zwischen 5 und 90 bar stromaufwärts des elektrisch beheizten Reformierungsreaktors,
- Durchleiten eines elektrischen Stroms durch die elektrische Heizeinheit, wodurch mindestens ein Teil des ersten Katalysators auf eine Temperatur von mindestens 450°C erhitzt wird,
wobei die elektrische Heizeinheit eine makroskopische Struktur aus elektrisch leitfähigem Material umfasst, wobei die makroskopische Struktur eine Keramikbeschichtung trägt und die Keramikbeschichtung den ersten Katalysator trägt.

14. Verfahren nach Anspruch 13, das ferner den Schritt der Zugabe eines oder mehrerer zusätzlicher Einsatzströme zum Reformierungsabschnitt stromaufwärts des elektrisch beheizten Reformers, zum ersten Synthesegas und/oder direkt zum autothermen Reformierungsreaktor umfasst.

15. Verfahren nach Anspruch 14, worin ein Endgas aus einer Fischer-Tropsch-Einheit dem ersten Synthesegas oder direkt dem autothermen Reformierungsreaktor zugesetzt wird.

16. Verfahren nach einem der Ansprüche 13 bis 15, das ferner den Schritt des Vorreformierens des Einsatzgases in einem Vorreformer stromaufwärts des elektrisch beheizten Reformierungsreaktors umfasst.

17. Verfahren nach einem der Ansprüche 13 bis 16, wobei der Reformierungsabschnitt ferner einen gasbeheizten Dampf-Methan-Reformierungsreaktor parallel zu dem elektrisch beheizten Reformierungsreaktor und dem autothermen Reformierungsreaktor umfasst, wobei der gasbeheizte Dampf-Methan-Reformierungsreaktor einen dritten Katalysator umfasst, wobei das Verfahren ferner die Schritte umfasst:
- Einführen eines zweiten Einsatzmaterials, umfassend Kohlenwasserstoffe, in den gasbeheizten Dampf-Methan-Reformierungsreaktor,
- Nutzung mindestens eines Teils des zweiten Synthesegases als Heizmedium im Wärmeaustausch innerhalb des gasbeheizten Dampf-Methan-Reform ierungsreaktors,
- Erzeugen eines dritten Synthesegases über dem dritten Katalysator innerhalb des gasbeheizten Dampf-Methan-Reformierungsreaktors, und
- Ausgeben des dritten Synthesegases aus dem Reformierungsabschnitt als mindestens ein Teil des Ausgabesynthesegases.

18. Verfahren nach einem der Ansprüche 13 bis 17, ferner umfassend:
- Nachbearbeitung des Ausgabesynthesegases in einer Nachbearbeitungseinheit stromabwärts des Reformierungsabschnitts, um ein nachbearbeitetes Synthesegas bereitzustellen.

19. Verfahren nach Anspruch 18, wobei die Nachbearbeitungseinheit eine Nachkonvertierungseinheit ist, in der ein vierter Katalysator untergebracht ist, der aktiv ist für das Katalysieren von Dampf-Methan-Reformierung, Methanisierung und reversen Wassergas-Shift-Reaktionen, wobei das Verfahren zudem den Schritt des Einführens von erhitztem CO₂ in das Ausgabesynthesegas stromaufwärts der Nachkonvertierungseinheit umfasst.

20. Verfahren nach Anspruch 18 oder 19, wobei die Nachbearbeitungseinheit eine Wassergas-Shift-Einheit ist und der Schritt der Nachbearbeitung des Ausgabesynthesegases die Durchführung der Wassergas-Shift-Reaktion umfasst.

21. Verfahren nach einem der Ansprüche 13 bis 20, ferner umfassend den Schritt:
- Trennen des Ausgabesynthesegases oder des nachbearbeiteten Synthesegases in ein Wasserkondensat und ein intermediäres Synthesegas in einer ersten Trenneinheit stromabwärts der Nachbearbeitungseinheit.

22. Verfahren nach Anspruch 21, das ferner den folgenden Schritt umfasst:
- Bereitstellen des intermediären Synthesegases in einen stromabwärts gelegenen Abschnitt, der zur Aufnahme des intermediären Synthesegases und zur Verarbeitung des intermediären Synthesegases zu dem chemischen Produkt und einem Abgas eingerichtet ist.

23. Verfahren nach Anspruch 22, ferner umfassend:
- Bereitstellung von Brennstoff für eine befeuerte Heizeinheit stromaufwärts des autothermen Reformierungsreaktors, wobei die befeuerte Heizeinheit so betrieben werden kann, dass sie das Einsatzgas vorwärmt, und
- Rückführung mindestens eines Teils des Abgases aus dem stromabwärts gelegenen Abschnitt als Brennstoff in die befeuerte Heizeinheit.

24. Verfahren nach einem der Ansprüche 21 bis 23, wobei das Verfahren die Abtrennung eines Stroms von im Wesentlichen reinem CO₂, H₂ und/oder CO von dem intermediären Synthesegas in einer oder mehreren Gastrenneinheiten des stromabwärts gelegenen Abschnitts umfasst, wodurch ein raffiniertes Synthesegas bereitgestellt wird.

25. Verfahren nach einem der Ansprüche 21 bis 24, wobei das Verfahren ferner umfasst: Umwandeln des intermediären Synthesegases in Ammoniak in einem Ammoniakreaktor des stromabwärts gelegenen Abschnitts, Umwandeln des intermediären Synthesegases in Methanol in einem Methanolreaktor des stromabwärts gelegenen Abschnitts oder Umwandeln des intermediären Synthesegases in ein Gemisch höherer Kohlenwasserstoffe in einem Fischer-Tropsch-Reaktor.

## Revendications

1. Installation chimique comprenant:
- une section de reformage agencée pour recevoir un gaz d'alimentation qui comprend des hydrocarbures y compris du méthane et pour procurer un gaz de synthèse de sortie, dans laquelle ladite section de reformage comprend:
- un réacteur de reformage chauffé par voie électrique dans lequel est logé un premier catalyseur, ledit réacteur de reformage chauffé par voie électrique étant agencé pour recevoir ledit gaz d'alimentation et pour générer un premier gaz de synthèse par l'intermédiaire d'une réaction de production de méthane à la vapeur;
- un réacteur de reformage autothermique en aval dudit réacteur de reformage chauffé par voie électrique, un deuxième catalyseur étant logé dans ledit réacteur de reformage autothermique, ledit réacteur de reformage autothermique étant agencé pour recevoir ledit premier gaz de synthèse et pour produire un deuxième gaz de synthèse, dans laquelle ladite section de reformage est agencée pour produire ledit gaz de synthèse de sortie comprenant ledit deuxième gaz de synthèse;
dans laquelle ledit réacteur de reformage chauffé par voie électrique comprend:
- une enceinte sous pression dans laquelle est logée une unité de chauffage électrique agencée pour chauffer ledit premier catalyseur, dans laquelle ledit premier catalyseur peut être opéré pour catalyser le reformage à la vapeur dudit gaz d'alimentation, dans laquelle ladite enceinte sous pression possède une pression de consigne entre 5 et 90 bar;
- une couche d'isolation thermique adjacente à au moins une partie du côté interne de ladite enceinte sous pression; et
- au moins deux conducteurs reliés par voie électrique à ladite unité de chauffage électrique et à une alimentation en électricité placée à l'extérieur de ladite enceinte sous pression;
dans laquelle ladite alimentation en électricité est dimensionnée pour chauffer au moins une partie dudit premier catalyseur jusqu'à une température d'au moins 450°C en faisant circuler un courant électrique à travers ladite unité de chauffage électrique;
dans laquelle ladite unité de chauffage électrique comprend une structure macroscopique d'un matériau électroconducteur, ladite structure macroscopique supportant un revêtement en céramique et ledit revêtement en céramique supportant ledit premier catalyseur.

2. Installation chimique selon la revendication 1, comprenant en outre un préreformeur en amont dudit réacteur de reformage chauffé par voie électrique.

3. Installation chimique selon l'une quelconque des revendications 1 à 2, dans laquelle ladite section de reformage comprend en outre un réacteur de reformage du méthane à la vapeur chauffé au gaz monté en parallèle audit réacteur de reformage chauffé par voie électrique et audit réacteur de reformage autothermique, dans laquelle ledit réacteur de reformage du méthane à la vapeur chauffé au gaz comprend un troisième catalyseur et peut être opéré pour recevoir un deuxième gaz d'alimentation comprenant des hydrocarbures et pour utiliser au moins une partie dudit deuxième gaz de synthèse comme milieu de chauffage dans un échange thermique au sein dudit réacteur de reformage du méthane à la vapeur chauffé au gaz, ledit réacteur de reformage du méthane à la vapeur chauffé au gaz étant agencé pour générer un troisième gaz de synthèse.

4. Installation chimique selon l'une quelconque des revendications 1 à 3, comprenant en outre:
- une unité de traitement ultérieur en aval de la section de reformage, dans laquelle ladite unité de traitement ultérieur est agencée pour recevoir ledit gaz de synthèse de sortie et pour procurer un gaz de synthèse soumis à un traitement ultérieur.

5. Installation chimique selon la revendication 4, dans laquelle ladite unité de traitement ultérieur représente une unité de conversion ultérieure possédant une entrée pour permettre l'addition de CO₂ chauffé audit gaz de synthèse de sortie en amont de l'unité de conversion ultérieure, et dans laquelle est logé un quatrième catalyseur actif pour catalyser le reformage du méthane à la vapeur, la méthanation et la conversion du gaz à l'eau inverse.

6. Installation chimique selon la revendication 4 ou 5, dans laquelle ladite unité de traitement ultérieur représente une unité de conversion du gaz à l'eau agencée pour mettre en oeuvre la réaction de conversion du gaz à l'eau, pour ainsi procurer un gaz de synthèse soumis à un traitement ultérieur.

7. Installation chimique selon l'une quelconque des revendications 1 à 6, comprenant en outre:
- une première unité de séparation agencée pour séparer ledit gaz de synthèse de sortie ou ledit gaz de synthèse soumis à un traitement ultérieur en un condensat de vapeur d'eau et en un gaz de synthèse intermédiaire.

8. Installation chimique selon la revendication 7, comprenant en outre:
- une section en aval agencée pour recevoir le gaz de synthèse intermédiaire et pour traiter le gaz de synthèse intermédiaire afin d'obtenir un produit chimique et un gaz d'échappement.

9. Installation chimique selon la revendication 8, comprenant en outre:
- une unité sous la forme d'un dispositif de chauffage à combustible en amont dudit réacteur de reformage chauffé par voie électrique, l'unité sous la forme d'un dispositif de chauffage à combustible étant agencée pour préchauffer ledit gaz d'alimentation; et
- un moyen pour recycler au moins une partie dudit gaz d'échappement, à partir de ladite section en aval, sous forme de combustible en direction de l'unité sous la forme d'un dispositif de chauffage à combustible.

10. Installation chimique selon l'une quelconque des revendications 8 à 9, dans laquelle ladite section en aval comprend une ou plusieurs unités de séparation de gaz agencée(s) pour séparer un courant de CO₂, de H₂ et/ou de CO essentiellement pur(s) à partir dudit gaz de synthèse intermédiaire, pour ainsi procurer un gaz de synthèse épuré.

11. Installation chimique selon l'une quelconque des revendications 8 à 10, dans laquelle ladite section en aval comprend un réacteur de production d'ammoniac destiné à convertir en ammoniac ledit gaz de synthèse intermédiaire ou ledit gaz de synthèse épuré, un réacteur de production de méthanol destiné à convertir en méthanol ledit gaz de synthèse intermédiaire ou ledit gaz de synthèse épuré, ou un réacteur de Fischer-Tropsch destiné à convertir en un mélange d'hydrocarbures supérieurs ledit gaz de synthèse intermédiaire ou ledit gaz de synthèse épuré.

12. Installation chimique selon l'une quelconque des revendications 1 à 11, dans laquelle l'alimentation en électricité et l'unité de chauffage électrique au sein de l'enceinte sous pression sont dimensionnées d'une manière telle qu'au moins une partie de l'unité de chauffage électrique atteint une température de 450°C à 850°C, telle que de 500°C à 750°C, et de préférence de 550°C à 650°C.

13. Procédé pour la production d'un produit chimique à partir d'un gaz d'alimentation comprenant des hydrocarbures y compris du méthane, dans une installation chimique comprenant une section de reformage, ladite section de reformage comprenant un réacteur de reformage chauffé par voie électrique dans laquelle est logé un premier catalyseur, et un réacteur de reformage autothermique en aval dudit réacteur de reformage chauffé par voie électrique, un deuxième catalyseur étant logé dans ledit réacteur de reformage autothermique, ledit procédé comprenant les étapes dans lesquelles:
- on introduit ledit gaz d'alimentation dans ledit réacteur de reformage chauffé par voie électrique et on met en oeuvre un reformage du méthane à la vapeur afin d'obtenir un premier gaz de synthèse;
- on introduit ledit premier gaz de synthèse dans ledit réacteur de reformage autothermique, et on met en oeuvre un reformage du méthane à la vapeur afin d'obtenir un deuxième gaz de synthèse;
- on évacue un gaz de synthèse qui comprend ledit deuxième gaz de synthèse à partir de ladite section de reformage;
dans lequel ledit réacteur de reformage chauffé par voie électrique comprend une enceinte sous pression dans laquelle est logée une unité de chauffage électrique agencée pour chauffer ledit premier catalyseur, dans lequel ledit premier catalyseur peut être opéré pour catalyser le reformage à la vapeur dudit gaz d'alimentation, dans lequel ladite enceinte sous pression possède une pression de consigne entre 5 et 90 bar;
- une couche d'isolation thermique adjacente à au moins une partie du côté interne de ladite enceinte sous pression; et
- au moins deux conducteurs reliés par voie électrique à ladite unité de chauffage électrique et à une alimentation en électricité placée à l'extérieur de ladite enceinte sous pression;
dans lequel ledit procédé comprend en outre les étapes dans lesquelles:
- on met sous pression ledit gaz d'alimentation jusqu'à une pression entre 5 et 90 bar en amont dudit réacteur de reformage chauffé par voie électrique;
- on fait circuler un courant électrique à travers ladite unité de chauffage électrique pour ainsi chauffer au moins une partie dudit premier catalyseur jusqu'à une température d'au moins 450°C;
dans lequel ladite unité de chauffage électrique comprend une structure macroscopique d'un matériau électroconducteur, ladite structure macroscopique supportant un revêtement en céramique et ledit revêtement en céramique supportant ledit premier catalyseur.

14. Procédé selon la revendication 13, comprenant en outre l'étape dans laquelle on ajoute un ou plusieurs courants d'alimentation supplémentaires à la section de reformage en amont du reformeur chauffé par voie électrique, au premier gaz de synthèse et/ou directement au réacteur de reformage autothermique.

15. Procédé selon la revendication 14, dans lequel on ajoute un gaz résiduaire à partir d'une unité de Fischer-Tropsch au premier gaz de synthèse ou directement au réacteur de reformage autothermique.

16. Procédé selon l'une quelconque des revendications 13 à 15, comprenant en outre l'étape dans laquelle on soumet ledit gaz d'alimentation à un préreformage dans un préreformeur en amont dudit réacteur de reformage chauffé par voie électrique.

17. Procédé selon l'une quelconque des revendications 13 à 16, dans lequel ladite section de reformage comprend en outre un réacteur de reformage du méthane à la vapeur chauffé au gaz monté en parallèle audit réacteur de reformage chauffé par voie électrique et audit réacteur de reformage autothermique, dans lequel ledit réacteur de reformage du méthane à la vapeur chauffé au gaz comprend un troisième catalyseur, ledit procédé comprenant en outre les étapes dans lesquelles:
- on introduit une deuxième alimentation comprenant des hydrocarbures dans ledit réacteur de reformage du méthane à la vapeur chauffé au gaz;
- on utilise au moins une partie dudit deuxième gaz de synthèse comme milieu de chauffage dans un échange thermique au sein dudit réacteur de reformage du méthane à la vapeur chauffé au gaz;
- on génère un troisième gaz de synthèse par-dessus le troisième catalyseur au sein du réacteur de reformage du méthane à la vapeur chauffé au gaz; et
- on évacue ledit troisième gaz de synthèse à partir de ladite section de reformage sous la forme d'au moins une partie dudit gaz de synthèse de sortie.

18. Procédé selon l'une quelconque des revendications 13 à 17, comprenant en outre le fait de:
- dans une unité de traitement ultérieur en aval de ladite section de reformage, soumettre à un traitement ultérieur ledit gaz de synthèse de sortie afin d'obtenir un gaz de synthèse soumis à un traitement ultérieur.

19. Procédé selon la revendication 18, dans lequel ladite unité de traitement ultérieur représente une unité de conversion ultérieure dans laquelle est logé un quatrième catalyseur actif pour catalyser des réactions de reformage du méthane à la vapeur, de méthanation et de conversion du gaz à l'eau inverse, dans lequel ledit procédé comprend en outre l'étape dans laquelle on introduit du CO₂ chauffé dans ledit gaz de synthèse de sortie en amont de ladite unité de conversion ultérieure.

20. Procédé selon la revendication 18 ou 19, dans lequel ladite unité de traitement ultérieur représente une unité de conversion du gaz à l'eau et l'étape dans laquelle on soumet à un traitement ultérieur ledit gaz de synthèse de sortie comprend la mise en oeuvre de la réaction de conversion du gaz à l'eau.

21. Procédé selon l'une quelconque des revendications 13 à 20, comprenant en outre l'étape dans laquelle:
- on sépare ledit gaz de synthèse de sortie ou ledit gaz de synthèse soumis à un traitement ultérieur en un condensat de vapeur d'eau et en un gaz de synthèse intermédiaire dans une première unité de séparation en aval de ladite unité de traitement ultérieur.

22. Procédé selon la revendication 21, comprenant en outre l'étape dans laquelle:
- on procure ledit gaz de synthèse intermédiaire à une section en aval agencée pour recevoir le gaz de synthèse intermédiaire et pour traiter le gaz de synthèse intermédiaire afin d'obtenir ledit produit chimique et un gaz d'échappement.

23. Procédé selon la revendication 22, comprenant en outre le fait de:
- procurer un combustible à une unité de chauffage à combustible en amont dudit réacteur de reformage autothermique, ladite unité sous la forme d'un dispositif de chauffage à combustible pouvant être opérée pour préchauffer ledit gaz d'alimentation; et
- recycler sous forme de combustible au moins une partie dudit gaz d'échappement, à partir de ladite section en aval, en direction de l'unité sous la forme d'un dispositif de chauffage à combustible.

24. Procédé selon l'une quelconque des revendications 21 à 23, dans lequel ledit procédé comprend le fait de séparer un courant de CO₂, de H₂ et/ou de CO essentiellement pur(s) à partir dudit gaz de synthèse intermédiaire, pour ainsi procurer un gaz de synthèse épuré, dans une ou plusieurs unité(s) de séparation de gaz de ladite section en aval.

25. Procédé selon l'une quelconque des revendications 21 à 24, dans lequel ledit procédé comprend en outre le fait de:
- convertir en ammoniac ledit gaz de synthèse intermédiaire dans un réacteur de production d'ammoniac de ladite section en aval, convertir en méthanol ledit gaz de synthèse intermédiaire dans un réacteur de production de méthanol de ladite section en aval ou convertir ledit gaz de synthèse intermédiaire en un mélange d'hydrocarbures supérieurs dans un réacteur de Fischer-Tropsch.
